# EUROPEAN PATENT APPLICATION

(11) **EP 4 691 638 A1**
(43) Date of publication of application: **11.02.2026**
(21) Application number: 24779877.0
(22) Date of filing: 21.03.2024
(51) Int. Cl.: B01J 35/50, B01J 23/83, B01J 35/57, C07B 61/00, C07C 1/12, C07C 9/04

(54) **HONEYCOMB STRUCTURE, METHANE PRODUCTION DEVICE, AND METHANE PRODUCTION METHOD**

(30) Priority: 31.03.2023 JP 2023057140
(71) Applicant: NGK Insulators, Ltd., Nagoya-shi, Aichi 467-8530 (JP)
(72) Inventor: MAEDA Kazuki, Nagoya-shi, Aichi 467-8530 (JP); HORI Keigo, Nagoya-shi, Aichi 467-8530 (JP); KOBAYASHI Yoshimasa, Nagoya-shi, Aichi 467-8530 (JP); KATSUDA Yuji, Nagoya-shi, Aichi 467-8530 (JP)
(74) Representative: Müller-Boré & Partner Patentanwälte PartG mbB
(86) International application number: PCT/JP2024/011125
(87) International publication number: WO 2024/203758

(57) **Abstract**

Provided is a honeycomb structural body, a methane production apparatus, and a method of producing methane, which enable a reduction in size of the methane production apparatus and production of methane in an energy-saving manner. A honeycomb structural body according to an embodiment of the present disclosure includes a honeycomb-like base material and catalyst layers. The honeycomb-like base material includes a partition wall that defines a plurality of cells. The cells each include a gas flow passage. The catalyst layers contain a methanation reaction catalyst for methanation of carbon dioxide through a reaction with hydrogen. The catalyst layers are each provided on a surface of the partition wall so as to face the gas flow passages, respectively. The honeycomb-like base material includes a heat-generating portion capable of generating heat by application of a voltage.

## Description

### Technical Field

The present disclosure relates to a honeycomb structural body, a methane production apparatus, and a method of producing methane.

### Background Art

In recent years, capture of carbon dioxide and its reuse as a raw material for a carbon compound have been under consideration in terms of a reduction in environmental load. For example, methanation for converting carbon dioxide to methane through a reaction with hydrogen has been proposed (see, for example, Patent Literature 1). However, such methanation requires heating of a reactor to a predetermined temperature or higher so as to start a methanation reaction. Thus, a methane production apparatus for performing methanation is required to include a heating device for heating the reactor, leading to a problem in that the methane production apparatus is increased in size.

### Citation List

### Patent Literature

[PTL 1] JP 2015-196619 A

### Summary of Invention

### Technical Problem

A primary object of the present disclosure is to provide a honeycomb structural body, a methane production apparatus, and a method of producing methane, which enable a reduction in size of the methane production apparatus and production of methane in an energy-saving manner.

### Solution to Problem

[1] According to one embodiment of the present disclosure, there is provided a honeycomb structural body including a honeycomb-like base material and catalyst layers. The honeycomb-like base material includes a partition wall that defines a plurality of cells. The cells each include a gas flow passage. The catalyst layers contain a methanation reaction catalyst for methanation of carbon dioxide through a reaction with hydrogen. The catalyst layers are each provided on a surface of the partition wall so as to face the gas flow passages, respectively. The honeycomb-like base material includes a heat-generating portion capable of generating heat by application of a voltage.
[2] In the honeycomb structural body according to the above-mentioned item [1], the heat-generating portion may be formed of an electrically conductive ceramic material.
[3] In the honeycomb structural body according to the above-mentioned item [2], the electrically conductive ceramic material may contain Si and SiC.
[4] In the honeycomb structural body according to the above-mentioned item [3], an oxide coating may be formed on a surface of a combination of Si and SiC.
[5] In the honeycomb structural body according to any one of the above-mentioned items [1] to [4], the honeycomb-like base material may further include an insulating portion formed of an insulating ceramic material. The heat-generating portion may be positioned on an upstream side of the insulating portion in a direction of passage of a fluid through the gas flow passages.
[6] According to another aspect of the present disclosure, there is provided a methane production apparatus, including the honeycomb structural body of any one of the above-mentioned items [1] to [5].
[7] According to still another aspect of the present disclosure, there is provided a method of producing methane, including: a step of causing the heat-generating portion of the honeycomb-like base material included in the honeycomb structural body of any one of the above-mentioned items [1] to [5] to generate heat at 200°C or more and 500°C or less by applying a voltage to the heat-generating portion; and a step of supplying a raw material gas containing carbon dioxide and hydrogen to the gas flow passages after the heat-generating portion generates heat.

### Advantageous Effects of Invention

According to the embodiments of the present disclosure, the honeycomb structural body, the methane production apparatus, and the method of producing methane, which enable a reduction in size of the methane production apparatus and production of methane in an energy-saving manner, can be achieved.

### Brief Description of Drawings

FIG. **1** is a schematic perspective view of a honeycomb structural body according to one embodiment of the present disclosure.
FIG. **2** is a schematic sectional view of the honeycomb structural body of FIG. **1****.**
FIG. **3** is a schematic sectional view of a honeycomb structural body according to another embodiment of the present disclosure.

### Description of Embodiments

Embodiments of the present disclosure are described below with reference to the drawings. However, the present disclosure is not limited to these embodiments. In addition, in the drawings, the width, thickness, shape, and the like of each portion may be schematically illustrated as compared to those in the embodiments in order to provide clearer description, but the drawings are merely examples and do not limit the interpretation of the present disclosure.

### A. Overview of Honeycomb Structural Body

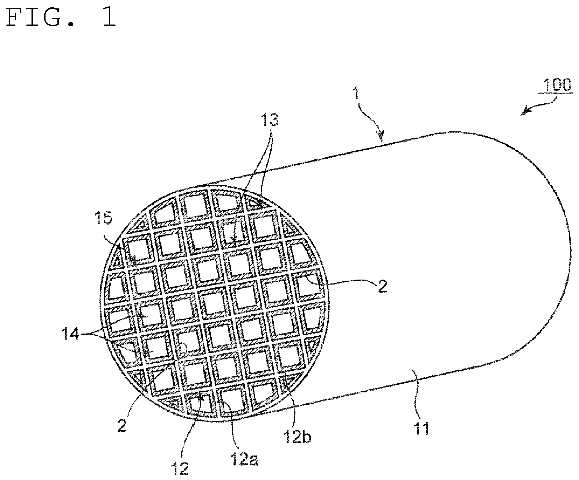

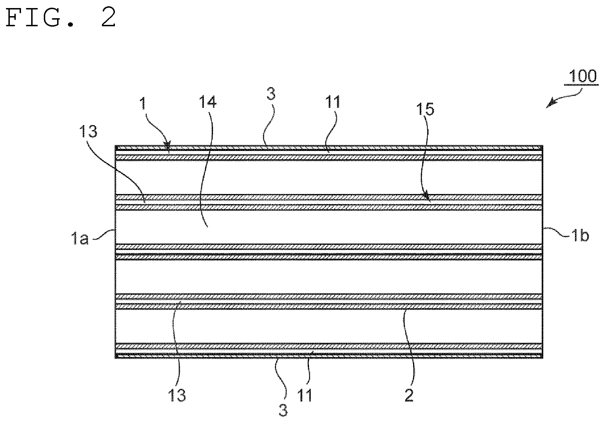

FIG. **1** is a schematic perspective view of a honeycomb structural body according to one embodiment of the present disclosure, and FIG. **2** is a schematic sectional view of the honeycomb structural body of FIG. **1****.**

A honeycomb structural body **100** of the illustrated example includes a honeycomb-like base material **1** and catalyst layers **2.** The honeycomb-like base material **1** includes a partition wall **12** that defines a plurality of cells **13.** The cells **13** each include a gas flow passage **14.** The catalyst layers **2** contain a methanation reaction catalyst for methanation of carbon dioxide through a reaction with hydrogen. The catalyst layers **2** are formed on a surface of the partition wall **12** so as to face the gas flow passages **14,** respectively. The honeycomb-like base material **1** includes a heat-generating portion **15** that is capable of generating heat by the application of a voltage.

In the configuration as described above, the honeycomb-like base material includes the heat-generating portion. Thus, when a voltage is applied to the heat-generating portion, the honeycomb structural body (in particular, the catalyst layers) can be heated to a temperature at which a methanation reaction expressed by the following Formula (1) is started or higher without additionally providing a heating device. Thus, using the honeycomb structural body as described above for a methane production apparatus enables a reduction in size of the methane production apparatus and a reduction in facility costs. Further, the partition wall of the honeycomb-like base material defines the plurality of cells, and the catalyst layers are formed on the surface of the partition wall. Thus, when carbon dioxide and hydrogen are caused to pass through the gas flow passages, and are supplied to the catalyst layers, carbon dioxide and hydrogen can be efficiently brought into contact with the methanation reaction catalyst, allowing the methanation reaction expressed by the following Formula (1) to smoothly progress. The methanation reaction (1) is an exothermic reaction. Thus, generated heat (reaction heat) can be effectively utilized for the progress of the chemical reaction. As a result, methane can be produced in an energy-saving manner.

CO₂+4H₂→CH₄+2H₂O (1)

### B. Details of Honeycomb-Like Base Material

The honeycomb-like base material **1** typically includes a flow-through type honeycomb structure. The honeycomb-like base material **1** has any appropriate shape (overall shape). The shape of the honeycomb-like base material **1** is, for example, a cylinder with a circle as its bottom, an elliptic cylinder with an ellipse as its bottom, a prismatic column with a polygon as its bottom, or a column with an indefinite shape as its bottom. In one embodiment, the honeycomb-like base material **1** has a columnar shape. The outer diameter and length of the honeycomb-like base material **1** may be appropriately set in accordance with purposes. The honeycomb-like base material **1** may have a hollow region in a center portion thereof in the cross section in the direction perpendicular to the lengthwise direction, though the hollow region is not shown.

In the illustrated example, the honeycomb-like base material **1** includes: an outer peripheral wall **11;** and a partition wall **12** positioned inside the outer peripheral wall **11.** The outer peripheral wall **11** and the partition wall **12** may be integrally formed or may be separate bodies. In the illustrated example, the outer peripheral wall **11** and the partition wall **12** are integrally formed.

The outer peripheral wall **11** has a cylindrical shape. The thickness of the outer peripheral wall **11** may be appropriately set in accordance with the application of the honeycomb structural body. The thickness of the outer peripheral wall **11** may be, for example, from 0.1 mm to 10 mm, or may be, for example, from 2 mm to 8 mm.

As described above, the partition wall **12** defines the plurality of cells **13.**

The cells **13** each extend from a first end surface **1a** (inflow end surface) of the honeycomb-like base material **1** to a second end surface **1b** (outflow end surface) thereof in the lengthwise direction (axial direction) of the honeycomb-like base material **1** (see FIG. **2**). The cells **13** each have any appropriate shape in a cross section in a direction perpendicular to the lengthwise direction of the honeycomb structural body **1.** The sectional shapes of the cells are each, for example, a triangle, a quadrangle, a pentagon, a hexagon, a higher polygon, a circle, or an ellipse. The sectional shapes and sizes of the cells may be all the same, or may be at least partly different. Of such sectional shapes of the cells, for example, a quadrangle is preferred, and a square or a rectangle is more preferred.

A cell density of the honeycomb-like base material **1** is, for example, 100 cpsi or more, preferably 300 cpsi or more. Meanwhile, the upper limit of the cell density of the honeycomb-like base material **1** is typically 1,000 cpsi. When the cell density falls within such range, carbon dioxide and hydrogen can be brought into contact with the catalyst layers in a more efficient manner. Thus, methane can be produced in an energy-saving manner at an excellent methane conversion rate.

The phrase "cell density of the honeycomb-like base material" as used herein refers to a cell density in a cross section in a lengthwise direction (direction in which the cells extend) of the honeycomb-like base material, and the unit "cpsi" as used herein refers to the number of cells for 6.4516 cm² (per square inch) of the cross section.

The gas flow passage **14** is defined inside the cell **13,** which is described later in detail. The gas flow passage **14** is a space defined inside the cell **13,** and like the cell **13,** extends from the first end surface **1a** (inflow end surface) to the second end surface **1b** (outflow end surface). Examples of a sectional shape of each of the gas flow passages **14** include shapes similar to those of the cell **13** described above. Of the sectional shapes, a quadrangle is preferred, and a square or a rectangle is more preferred. The sectional shapes and sizes of the gas flow passages **14** may be all the same, or may be at least partly different.

In the illustrated example, the partition wall **12** has a first partition wall **12a** and a second partition wall **12b** perpendicular to each other, and the first partition wall **12a** and the second partition wall **12b** define the plurality of cells **13.** The sectional shapes of the cells **13** are each a quadrangle except for a portion in which the first partition wall **12a** and the second partition wall **12b** are each brought into contact with the outer peripheral wall **11.** The configuration of the partition wall is not limited to the partition wall **12** described above. The partition wall may have a first partition wall extending in a radial direction and a second partition wall extending in a circumferential direction, which define a plurality of cells.

The thickness of the partition wall **12** may be appropriately and suitably set. The thickness of the partition wall **12** is typically smaller than the thickness of the outer peripheral wall **11.** The thickness of the partition wall **12** is, for example, 0.0127 mm (0.5 mil) or more, preferably 0.0254 mm (1.0 mil) or more. Meanwhile, the thickness of the partition wall **12** is, for example, 0.508 mm (20 mil) or less, preferably 0.254 (10 mil) mm or less, more preferably 0.2032 (8.0 mil) mm or less, still more preferably 0.127 (5.0 mil) mm or less. When the thickness of the partition wall falls within such ranges, the honeycomb structural body can achieve sufficient mechanical strength, and the cell density can be adjusted to fall within the above-mentioned ranges. The thickness of the partition wall is measured, for example, through sectional observation with a scanning electron microscope (SEM).

The partition wall **12** typically has a plurality of pores.

A mean pore diameter of the partition wall **12** may be appropriately set in accordance with purposes. The mean pore diameter of the partition wall **12** is, for example, 1 µm or more, preferably 5 µm or more. Meanwhile, the mean pore diameter of the partition wall **12** is, for example, 20 µm or less, preferably 15 µm or less. The mean pore diameter may be measured, for example, by mercury porosimetry.

A porosity of the partition wall **12** may be appropriately set in accordance with purposes. The porosity of the partition wall **12** is, for example, 15% or more, preferably 30% or more, more preferably 35% or more, still more preferably 40% or more. Meanwhile, the porosity of the partition wall **12** is, for example, 60% or less, preferably 55% or less, more preferably 50% or less. The porosity may be measured, for example, by mercury porosimetry.

When the mean pore diameter and/or the porosity of the partition wall **12** falls within such ranges, the amount of the methanation reaction catalyst supported in the partition wall can be improved.

A density of the partition wall **12** may be appropriately set in accordance with purposes. The density of the partition wall **12** is, for example, from 1.7 g/cm³ to 2.8 g/cm³, preferably from 1.8 g/cm³ to 2.6 g/cm³. The density is measured, for example, by mercury porosimetry.

As described above, the honeycomb-like base material **1** includes at least the heat-generating portion **15.**

The heat-generating portion **15** may be the entirety of the honeycomb-like base material **1** or may be part of the honeycomb-like base material **1.** In FIG. **1** and FIG. **2****,** the entirety of the honeycomb-like base material **1** (that is, the outer peripheral wall **11** and the partition wall **12)** is formed as the heat-generating portion **15.**

Meanwhile, only part of the honeycomb-like base material **1** may be formed as the heat-generating portion **15.** For example, in the honeycomb-like base material **1,** the outer peripheral wall **11** may be formed as the heat-generating portion **15,** and the partition wall **12** may be formed as a thermally conductive portion to which heat from the outer peripheral wall **11** is transferred. Further, in the honeycomb-like base material **1,** for example, the partition wall **12** may be formed as the heat-generating portion **15,** and the outer peripheral wall **11** may be formed as an electrically conductive portion that is electrically connected to the partition wall **12.**

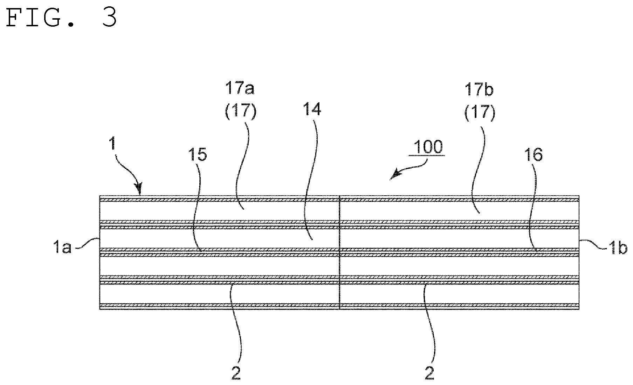

As illustrated in FIG. **3****,** in one embodiment, the honeycomb-like base material **1** further includes an insulating portion **16** in addition to the heat-generating portion **15.** The heat-generating portion **15** is positioned on an upstream side of the insulating portion **16** in a direction of passage of a fluid through the gas flow passages **14.** In the production of methane, a fluid passing through the honeycomb structural body is heated with the reaction heat described above, and the heated fluid transfers heat to a downstream part. Thus, the downstream part of the honeycomb structural body also has a high temperature. In the configuration described above, the heat-generating portion is positioned on the upstream side of the insulating portion. Thus, the honeycomb structural body can be efficiently heated from its upstream part to its downstream part. Accordingly, during the production of methane, uniformity of a temperature of the honeycomb structural body can be improved, enabling the production of methane in a more energy-saving manner.

In the illustrated example, the honeycomb-like base material **1** is divided into a plurality of blocks **17** in the direction of passage of the fluid through the gas flow passages **14.** The honeycomb-like base material **1** includes a first block **17a** and a second block **17b.** The first block **17a** is positioned on an upstream side of the second block **17b** in the direction of passage of the fluid through the gas flow passages **14.** The first block **17a** typically has the first end surface **1a** (inflow end surface) of the honeycomb-like base material **1.** The second block **17b** typically has the second end surface **1b** (outflow end surface) of the honeycomb-like base material **1.**

The entirety of the first block **17a** is formed as the heat-generating portion **15,** and the entirety of the second block **17b** is formed as the insulating portion **16.**

A length of the first block **17a** may be appropriately and suitably adjusted. The length of the first block **17a** is, for example, from 0.1 times to 3.0 times, preferably from 0.8 times to 1.2 times a length of the second block **17b.**

The heat-generating portion **15** is typically formed of an electrically conductive ceramic material. A volume resistivity of the electrically conductive ceramic material at 25°C is, for example, 200 Ω·cm or less, preferably 50 Ω·cm or less, more preferably 10 Ω·cm or less, still more preferably 1.0 Ω·cm or less. Meanwhile, the volume resistivity of the electrically conductive ceramic material at 25°C is, for example, 0.001 Ω·cm or more, preferably 0.1 Ω·cm or more. When the volume resistivity of the electrically conductive ceramic material falls within such ranges, the heat-generating portion can stably generate heat under the application of a voltage (for example, 50 V) to the heat-generating portion.

Examples of the electrically conductive ceramic material include an electrically conductive zirconia-based material, an alumina-titanium carbide-based composite material, and a ceramic material containing Si and SiC (typically, Si-SiC-based composite ceramic material). The electrically conductive ceramic materials may be used alone or in combination thereof.

Of the electrically conductive ceramic materials, a Si-SiC-based composite ceramic material (hereinafter referred to as "Si-SiC-based composite material") is preferred. When the electrically conductive ceramic material contains Si and SiC, the improvement in heat generation performance of the heat-generating portion can be achieved.

The Si-SiC-based composite material may have any appropriate configuration. In the Si-SiC-based composite material, an oxide coating (specifically, an SO₂ coating) is preferably formed on a surface of a combination of Si and SiC. The oxide coating can improve durability.

The Si-SiC-based composite material may be a porous body or a dense body. The porous body of the Si-SiC-based composite material is described in detail in, for example, JP 2002-201082 A. The dense body of the Si-SiC-based composite material is described in detail in, for example, JP 11-035376 A. The entire descriptions of those publications are incorporated herein by reference.

The insulating portion **16** is typically formed of an insulating ceramic material. A volume resistivity of the insulating ceramic material at 25°C is, for example, more than 1.0×10¹¹ Ω·cm, preferably 1.0×10¹³ Ω·cm or more.

Examples of the insulating ceramic material include aluminum nitride, aluminum oxide, silicon nitride, silicon carbide, zirconia, cordierite, and mullite. The insulating ceramic materials may be used alone or in combination thereof.

A thermal conductivity of the insulating portion **16** at 25°C is, for example, 0.5 W/(m-K) or more, preferably 5 W/(m·K) or more, more preferably 20 W/(m·K) or more, still more preferably 50 W/(m·K) or more. The upper limit of the thermal conductivity of the insulating portion **16** at 25°C is typically 200 W/(m·K). The thermal conductivity is measured, for example, by a flash method. When the thermal conductivity of the insulating portion is equal to or more than the above-mentioned lower limit, the insulating portion can be sufficiently heated with heat from the heat-generating portion. Thus, in the production of methane, the uniformity of the temperature of the honeycomb structural body including the heat-generating portion and the insulating portion can be sufficiently ensured.

### C. Details of Catalyst Layer

The catalyst layers **2** are formed on the surface of the partition wall **12.** In the honeycomb structural body **1,** the gas flow passage **14** is formed in a portion (typically, a center portion) in a cross section of the cell **13** in which the catalyst layer **2** is not formed. The catalyst layer **2** may be formed on the entire inner surface of the partition wall **12** (that is, so as to surround the gas flow passage **14)** as in the illustrated example, or may be formed on part of the surface of the partition wall. When the catalyst layer **2** is formed on the entire inner surface of the partition wall **12,** the methane conversion rate can be stably improved.

As described above, the catalyst layers **2** contain the methanation reaction catalyst.

The methanation reaction catalyst typically contains an active component and a carrier that supports the active component.

The active component can promote the methanation reaction expressed by Formula (1) described above. An example of the active component is a metal element. More specific examples thereof include: alkali metal elements, such as K and Na; platinum group elements, such as Ir, Ru, and Rh; alkaline earth metal element such as Ca; and Ni. The metal elements may be incorporated in the methanation reaction catalyst alone or in combination thereof. Those metal elements may be incorporated in the methanation reaction catalyst in a metal state, may be incorporated in the methanation reaction catalyst as a metal salt, or may be incorporated in the methanation reaction catalyst as an oxide.

Of those metal elements, Ni is preferred, and Ni in a metallic state is more preferred.

An example of the carrier is a metal oxide. More specific examples thereof include: oxides of rare earth elements, such as cerium oxide and yttrium oxide; zirconium oxide; aluminum oxide; silicon oxide; and magnesium oxide. The carriers may be incorporated in the methanation reaction catalyst alone or in combination thereof. Of those carriers, oxides of rare earth elements are preferred, and cerium oxide is more preferred.

A content ratio of the active component is, for example, from 0.01 part by mass to 50 parts by mass, preferably from 1 part by mass to 20 parts by mass with respect to 100 parts by mass of the carrier. When the content ratio of the active component falls within such ranges, the methanation reaction can be stably promoted.

The methanation reaction catalyst may have any appropriate shape. The methanation reaction catalyst typically has a particulate shape. A particulate methanation reaction catalyst is hereinafter sometimes referred to as "catalyst particle". In one embodiment, the catalyst layers 2 each contain an aggregate in which a plurality of catalyst particles are gathered together. The aggregate of the plurality of catalyst particles may form mesopores in the catalyst layers **2.** The catalyst layers **2** may contain another component (for example, a binder or the like) in addition to the methanation reaction catalyst.

A content ratio of the methanation reaction catalyst in the catalyst layers **2** is, for example, 50 mass% or more, preferably 70 mass% or more, more preferably 80 mass% or more, still more preferably 90 mass% or more. Meanwhile, the upper limit of the content ratio of the methanation reaction catalyst in the catalyst layers **2** is typically 100 mass%. When the content ratio of the methanation reaction catalyst in the catalyst layers falls within such ranges, the methanation reaction can be more stably promoted.

The amount of methanation reaction catalyst supported per unit area of the partition wall **12** is, for example, 20 mg/cm² or less, preferably 10 mg/cm² or less. Meanwhile, the amount of methanation reaction catalyst supported per unit area of the partition wall **12** is, for example, 0.5 mg/cm² or more, preferably 3.0 mg/cm² or more, more preferably 6 mg/cm² or more. When the amount of methanation reaction catalyst supported per unit area falls within such ranges, the formation of a hot spot (a localized high temperature area) in the catalyst layers can be prevented. Thus, the methane conversion rate can be stably improved.

The average pore diameter in the catalyst layer **2** is, for example, from 0.2 µm to 10 µm, preferably from 1.0 µm to 5.0 µm.

The BET specific surface area of the catalyst layer **2** is, for example, from 10 m²/g to 300 m²/g, preferably from 50 m²/g to 200 m²/g, more preferably from 80 m²/g to 150 m²/g.

The thickness of the catalyst layer **2** is, for example, from 2.0 µm to 200 µm, preferably from 5.0 µm to 100 µm, more preferably from 10 µm to 80 µm.

### D. Electrodes

As illustrated in FIG. **2****,** in one embodiment, the honeycomb structural body **100** further includes electrodes **3.** Thus, a voltage from an external power source (not shown) can be efficiently applied to the heat-generating portion of the honeycomb-like base material through intermediation of the electrodes. The electrodes **3** may be arranged at any appropriate positions as long as the electrodes **3** are electrically connected to the heat-generating portion **15.** In the illustrated example, two electrodes **3** are provided directly on an outer peripheral surface of the outer peripheral wall **11,** and are arranged spaced apart from each other in a circumferential direction of the outer peripheral wall **11.** As a material for the electrodes, any appropriate metal may be adopted.

### E. Method of Manufacturing Honeycomb Structural Body

Next, a method of manufacturing the honeycomb structural body **100** is described. In one embodiment, the method of manufacturing the honeycomb structural body **100** includes: a step of preparing the honeycomb-like base material **1;** and a step of forming the catalyst layers **2** on the partition wall **12** of the honeycomb-like base material **1.**

The honeycomb-like base material **1** formed of an electrically conductive ceramic material other than the dense body of the Si-SiC-based composite material is produced, for example, by the following method. First, a binder and water or an organic solvent are added to the above-mentioned electrically conductive ceramic material and/or material powder containing a raw material for the electrically conductive ceramic material, as required. The resultant mixture is kneaded to provide a body, and the body is molded (typically extruded) into a desired shape. After that, the body is dried, and is then fired as required.

Further, the honeycomb-like base material **1** formed of the dense body of the Si-SiC-based composite material may be produced by any appropriate method. One example of a method of producing the honeycomb-like base material **1** formed of the dense body of the Si-SiC-based composite material is described below.

First, a binder and water or an organic solvent are added to material powder including SiC powder, as required. The resultant mixture is kneaded to provide a body, and the body is molded (typically extruded) into a desired shape and is then dried to prepare a honeycomb dried body. After that, a Si supply body is heated while being in contact with the honeycomb dried body so that the honeycomb dried body is impregnated with a molten metal containing Si. A heating temperature for the Si supply body and the honeycomb dried body is, for example, from 1,200°C to 1,600°C. Further, heating time for the Si supply body and the honeycomb dried body is, for example, from 1 hour to 10 hours.

Next, the catalyst layers **2** are formed on the partition wall **12.** A method of forming the catalyst layers **2** is not particularly limited, and any appropriate method may be adopted. In one embodiment, a step of forming the catalyst layers **2** includes: a step of preparing the catalyst particles; a step of preparing a catalyst slurry in which the catalyst particles are dispersed; and a step of applying the catalyst slurry onto the partition wall **12,** in the stated order.

In the step of preparing the catalyst particles, for example, a carrier dispersion liquid in which particles of the above-mentioned metal oxide (carrier) (hereinafter referred to as "metal oxide particles") are dispersed and a metal salt solution in which a salt of the above-mentioned metal element (active component) (hereinafter referred to as "metal salt") is dissolved are prepared.

For the preparation of the carrier dispersion liquid, the metal oxide particles are added to a dispersion medium and are agitated.

A primary particle diameter of the metal oxide particles is, for example, from 5 nm to 200 nm.

The amount of metal oxide particles to be added is, for example, from 0.5 part by mass to 25 parts by mass with respect to 100 parts by mass of the dispersion medium.

Any appropriate solvent in which the metal oxide particles are insoluble may be adopted as the dispersion medium. Examples of the dispersion medium include water and alcohols. The dispersion media may be used alone or in combination thereof. Of the dispersion media, water is preferred.

For the preparation of the metal salt solution, the metal salt is added to a solvent and is agitated.

Examples of the metal salt include a nitric acid salt, a chloride, and a bromide. Of those, a nitric acid salt is preferred, and nickel nitride is more preferred. The metal salts may be used alone or in combination thereof.

The amount of the metal salt to be added is, for example, from 1.0 part by mass to 30 parts by mass with respect to 100 parts by mass of the solvent.

Examples of the solvent include solvents similar to the dispersion media described above. The solvents can be used alone or in combination thereof. Of the solvents, the same solvent as the dispersion medium used for the carrier dispersion liquid is preferred.

Next, the metal salt solution is added to the carrier dispersion liquid and is agitated. As a result, a mixed liquid of the carrier dispersion liquid and the metal salt solution is prepared.

A mixture ratio of the carrier dispersion liquid and the metal salt solution may be appropriately and suitably adjusted so that the content ratio of the metal element (active component) in the methanation reaction catalyst to be produced falls within the above-mentioned ranges.

Next, the mixed liquid is heated while being agitated so that the dispersion medium and the solvent are evaporated. As a result, a solid is obtained. After that, the solid is heated.

A heating temperature for the solid is, for example, from 300°C to 700°C, preferably from 400°C to 600°C.

Heating time for the solid is, for example, from 0.5 hour to 8 hours, preferably from 2 hours to 4 hours.

The solid may be heated in the atmosphere or under a reducing atmosphere (typically, a hydrogen atmosphere). The solid is preferably heated in the atmosphere.

In this manner, methanation reaction catalyst particles (catalyst particles) are prepared.

A primary particle diameter of the catalyst particles is, for example, from 5 nm to 80 nm.

In the step of preparing the catalyst slurry, the catalyst particles are added to the dispersion medium and are agitated.

As the dispersion medium, any appropriate solvent in which the catalyst particles are insoluble may be adopted. Examples of the dispersion medium include water and alcohols. The dispersion media may be used alone or in combination thereof. Of the dispersion media, water is preferred.

In this manner, the catalyst particles are dispersed in the dispersion medium to thereby prepare the catalyst slurry.

A content ratio of the catalyst particles in the catalyst slurry is, for example, from 3.0 mass% to 30 mass%, preferably from 5.0 mass% to 20 mass%. When the content ratio of the catalyst particles in the catalyst slurry falls within such ranges, the catalyst slurry can be smoothly applied onto the partition wall and thus the catalyst layers can be stably formed.

Next, in the step of applying the catalyst slurry, the catalyst slurry is applied onto the partition wall **12** of the honeycomb-like base material **1** by any appropriate method. In one embodiment, the honeycomb-like base material **1** is immersed into the catalyst slurry. Thus, the catalyst slurry can be sufficiently applied onto the surface of the partition wall even by a simple method. The application of the catalyst slurry is not limited to that described above. For example, the catalyst slurry may be caused to flow through the cells **13** of the honeycomb-like base material **1.** This also enables the application of the catalyst slurry onto the partition wall.

Next, coating films of the catalyst slurry formed on the partition wall **12** are dried at any appropriate heating temperature as required. As a result, the catalyst layers **2** are formed on the surface of the partition wall **12.**

The step of applying the catalyst slurry and the step of drying the coating films, which have been described above, may be repeated a plurality of times until the thicknesses of the catalyst layers **2** fall within a desired range.

Through the above-mentioned steps, the honeycomb structural body **100** including the honeycomb-like base material **1** and the catalyst layers **2** is manufactured.

The honeycomb structural body **100** is subjected to a reduction treatment as required. The reduction treatment allows the active component contained in the catalyst layers to be reduced to a metallic state. In the reduction treatment, the honeycomb structural body is thermally treated under an H₂ atmosphere. A heating temperature is, for example, from 300°C to 600°C, preferably from 400°C to 500°C. Heating time is, for example, from 0.1 hour to 8 hours, preferably from 0.5 hour to 2 hours.

The method of manufacturing the honeycomb structural body **100** may further include a step of forming the electrodes **3** in addition to the step of preparing the honeycomb-like base material **1** and the step of forming the catalyst layers **2,** which have been described above. In the step of forming the electrodes **3,** the electrodes **3** are typically formed on the outer peripheral wall **11** of the honeycomb-like base material **1** by any appropriate method.

### F. Methane Production Apparatus and Method of Producing Methane

The above-mentioned honeycomb structural body **100** is a honeycomb structural body for a methanation reaction, and may suitably be used for a methane production apparatus that produces methane through the reaction between carbon dioxide and hydrogen.

The methane production apparatus includes at least the honeycomb structural body **100** described above. The methane production apparatus may include any appropriate component in addition to the honeycomb structural body **100.** Meanwhile, the honeycomb-like base material **1** of the honeycomb structural body **100** includes the heat-generating portion **15.** Thus, the methane production apparatus is typically devoid of a heating device for heating the honeycomb structural body **100.**

Next, the method of producing methane according to one embodiment is described.

The method of producing methane includes a heat generating step and a gas supply step.

In the heat generating step, a voltage (for example, from 20 V to 400 V) is applied to the heat-generating portion **15** of the honeycomb structural body **100** to thereby cause the heat-generating portion **15** to generate heat. A heat generating temperature of the heat-generating portion **15** is typically 200°C or more and 500°C or less. In this manner, the temperature of the entirety of the honeycomb structural body **100** (in particular, the catalyst layers **2)** is raised to a predetermined methanation reaction starting temperature. The methanation reaction starting temperature is, for example, from 200°C to 300°C.

Next, the gas supply step is carried out. In the gas supply step, after the heat generating step, a raw material gas containing carbon dioxide and hydrogen is supplied to the gas flow passages **14** of the honeycomb structural body **100.** As a result, the raw material gas is supplied to the catalyst layers **2** having the methanation reaction starting temperature to thereby start the methanation reaction expressed by Formula (1) described above.

The content ratio of carbon dioxide in the raw material gas is, for example, from 1.0 vol% to 20 vol%, preferably from 5.0 vol% to 15 vol%.

The content ratio of hydrogen in the raw material gas is, for example, from 4.0 vol% to 80 vol%, preferably from 20 vol% to 60 vol%.

The raw material gas may contain oxygen in addition to carbon dioxide and hydrogen. When the raw material gas contains oxygen, a chemical reaction expressed by the following Formula (2) can progress upon contact of hydrogen and oxygen with the above-mentioned methanation reaction catalyst. The chemical reaction (2) is an exothermic reaction. Thus, reaction heat generated can be effectively used for the progress of the methanation reaction expressed by Formula (1) described above.

O₂+2H₂→2H₂O (2)

The content ratio of oxygen in the raw material gas is, for example, 0 vol% or more, preferably 0.5 vol% or more, more preferably 1.0 vol% or more. Meanwhile, the content ratio of oxygen in the raw material gas is, for example, 10 vol% or less, preferably 5.0 vol% or less.

Further, the raw material gas may contain nitrogen as a residual part.

For a flow rate of such raw material gas, any appropriate value may be adopted. The flow rate of the raw material gas is, for example, from 500 mL/minute to 15,000 mL/minute.

When the raw material gas is supplied to the catalyst layers **2** having the methanation reaction starting temperature, and the methanation reaction expressed by Formula (1) described above is started, the methanation reaction can be continued by using the reaction heat. Thus, the application of the voltage to the heat-generating portion **15** may be stopped. Under a state in which the application of the voltage to the heat-generating portion **15** is stopped, the honeycomb structural body **100** can be maintained at, for example, from 300°C to 500°C with the reaction heat. That is, the use of the honeycomb structural body **100** enables auto-methanation. Thus, energy savings in the production of methane can be achieved. Further, the honeycomb structural body **100** can be maintained at a temperature suitable for the methanation reaction without providing an external heating device and/or a heat retaining material to the honeycomb structural body. Thus, the methane production apparatus can be designed compactly.

As a result of the methanation reaction, a methane-containing gas is continuously discharged through the gas flow passages **14** of the honeycomb structural body **100.**

The methane-containing gas contains at least methane. The methane-containing gas may contain a residual unreacted raw material gas.

The methane conversion rate in such production of methane is, for example, 65% or more, preferably 70% or more, more preferably 75% or more, still more preferably 80% or more. Meanwhile, the methane conversion rate is, for example, 100% or less, and is, for example, 90% or less.

### Examples

Now, the present disclosure is specifically described by way of Examples. However, the present disclosure is not limited by these Examples.

### <Example 1>

### <<Preparation of Honeycomb-like Base Material>>

6 parts by mass of methylcellulose as an organic binder, 2.5 parts by mass of a surfactant, and 24 parts by mass of water were mixed to 100 parts by mass of mixed powder obtained by mixing 80 parts by mass of SiC raw material powder and 20 parts by mass of metal Si powder, to obtain a body. Next, after the body was subjected to extrusion and was then dried, the body was calcinated in an oxidizing atmosphere at 550°C for 3 hours and then was fired in a non-oxidizing atmosphere at 1,450°C for 2 hours. In this manner, the honeycomb-like base material illustrated in FIG. **1** was prepared. The honeycomb-like base material had a columnar shape with a diameter of 20 nm and a length of 50 mm. The honeycomb-like base material was formed of a porous body of the Si-SiC-based composite material. The entirety of the honeycomb-like base material can function as the heat-generating portion.

The honeycomb-like base material included: the partition wall that defined the plurality of cells; and the outer peripheral wall that surrounded the partition wall. The sectional shapes of the cells were each a quadrangle. The cell density of the honeycomb-like base material was 300 cpsi, and the thickness of the partition wall was 0.0254 mm. The mean pore diameter of the partition wall was 20 µm, and the porosity of the partition wall was 50%.

### <<Preparation of Methanation Reaction Catalyst>>

After cerium oxide (IV) particles (manufactured by Daiichi Kigenso Kagaku Kogyo Co., Ltd) were introduced into distilled water, the distilled water was agitated under reduced pressure at room temperature (23°C) for 12 hours. In this manner, a metal-oxide-particle dispersion liquid was obtained. Further, a hexahydrate of nickel nitrate (II) was dissolved in distilled water to obtain a nickel nitrate aqueous solution. Next, the nickel nitrate aqueous solution was added to the metal-oxide-particle dispersion liquid, and was agitated at room temperature (23°C) for 2 hours. After that, a mixed liquid of the dispersion liquid and the aqueous solution was heated to 80°C while being agitated, to evaporate water. Next, a residual solid was heated at 500°C for 3 hours. In this manner, methanation reaction catalyst particles (hereinafter referred to as "catalyst particles") were obtained. The catalyst particles contained nickel oxide (NiO) and cerium oxide (IV) that supported nickel oxide (NiO). A Ni content in the methanation reaction catalyst particles was 10 parts by mass with respect to 100 parts by mass of cerium oxide.

### <<Preparation of Catalyst Layers>>

A catalyst slurry was prepared by dispersing the resultant catalyst particles in distilled water. The content ratio of the catalyst particles in the catalyst slurry was 10 mass%. Next, the honeycomb-like base material prepared as described above was immersed in the catalyst slurry under normal pressure (0.1 MPa) at room temperature (23°C) for 5 seconds. After that, the honeycomb-like base material was removed from the catalyst slurry. In this manner, the catalyst slurry was applied onto the surface of the partition wall. After that, the catalyst slurry applied onto the surface of the partition wall was heated at 100°C for 120 minutes to be dried. The above-mentioned immersion and drying were repeated to form the catalyst layers on the surface of the partition wall. The catalyst layers each contained the aggregate of the catalyst particles. The thicknesses of the catalyst layers were each 3 µm. The amount of catalyst particles supported per unit area of the partition wall was 0.6 mg/cm². As a result of the above-mentioned immersion and drying repeated five times, the thickness of each of the catalyst layers was 15 µm. The amount of catalyst particles supported per unit area of the partition wall was 3.0 mg/cm².

After that, electrodes formed of platinum were formed by printing on the outer peripheral surface of the honeycomb-like base material. Then, the honeycomb-like base material was thermally treated in an H₂ atmosphere at 500°C for 1 hour to thereby activate NiO as Ni through reduction.

Through the steps described above, the honeycomb structural body including the honeycomb-like base material, the catalyst layers, and the electrodes was formed.

### <Example 2>

A honeycomb structural body was formed in the same manner as in Example 1 except that the honeycomb-like base material formed of the porous body of the Si-SiC-based composite material was replaced by a honeycomb-like base material prepared in the following manner.

A body containing SiC powder was subjected to extrusion and was then dried to thereby prepare a honeycomb dried body. The honeycomb dried body had a size and a configuration similar to those of the honeycomb-like base material of Example 1.

Further, material powder containing Si powder was pressmolded and was then dried to obtain a Si supply body.

Next, the Si supply body was heated under a reducedpressure condition (200 Pa) at 1,500°C for 4 hours under a state of being in contact with the honeycomb dried body, to thereby impregnate the honeycomb dried body with a molten metal containing Si.

In this manner, a honeycomb-like base material formed of a dense body of the Si-SiC-based composite material was prepared. The entirety of the honeycomb-like base material can function as the heat-generating portion.

### <Comparative Example 1>

A honeycomb structural body was formed in the same manner as in Example 1 except that the body containing the SiC raw material powder and the metal Si powder was replaced by a body containing cordierite. The entirety of the honeycomb-like base material of the honeycomb structural body can function as the insulating portion.

### <Example 3>

One honeycomb structural body was formed by arranging the honeycomb structural body (first block) of Example 1 and the honeycomb structural body (second block) of Comparative Example 1 in series so that the gas flow passages of the blocks were in communication with each other. The first block was positioned on an upstream side of the second block in the direction of passage of the fluid through the gas flow passages.

### <Auto-methanation Test>

The honeycomb structural bodies obtained in Examples and Comparative Example were each inserted into a reaction tube with an inner diameter of 21 mm. After that, a voltage (310 V for Examples 1 and 3, and 40 V for Example 2) was applied across the electrodes formed on the outer peripheral surface of the honeycomb-like base material, to cause the heat-generating portion of the honeycomb-like base material to generate heat so that the temperature of the honeycomb structural body was raised to 200°C. Further, a mixed gas of carbon dioxide at 10 vol%, oxygen at 3 vol%, hydrogen at 46 vol%, and nitrogen for a residual part was introduced as a raw material gas into the reaction tube. The flow rate of the raw material gas introduced into the reaction tube was set to 1,000 mL/minute.

The application of the voltage across the electrodes was stopped while the raw material gas was kept flowing through the reaction tube. The temperature of the honeycomb structural body was maintained at about 400°C with the reaction heat even after the application of the voltage was stopped.

As a result, the raw material gas passed through the gas flow passages of the honeycomb structural body, and a methane-containing gas flowed out from the reaction tube. A concentration of carbon dioxide and a concentration of methane in the methane-containing gas that had flowed out from the reaction tube was measured with a gas chromatography-thermal conductivity detector (GC-TCD). From the results of measurement, the viability of auto-methanation was evaluated based on the following criterion.
Viable: Generation of methane was confirmed after the application of the voltage was stopped.
Unviable: Generation of methane was not confirmed after the application of the voltage was stopped.

**[Table 1]**

| No. | Partition wall | | Application of voltage | Viability of auto-methanation |
|---|---|---|---|---|
| | Upstream part | Downstream part | | |
| | Material | Material | | |
| Example 1 | Porous body of Si-SiC-based composite | - | With | Viable |
| Example 2 | Dense body of Si-SiC-based composite | - | With | Viable |
| Example 3 | Porous body of Si-SiC-based composite | Cordierite | With | Viable |
| Comparative Example 1 | Cordierite | - | With | Unviable |

### <Evaluation>

As shown in Table 1, it is understood that, when the honeycomb-like base material includes the heat-generating portion, the methanation reaction can be started without a heating device for heating the honeycomb structural body from outside, and can be continued in an energy-saving manner.

### Industrial Applicability

The honeycomb structural body according to the embodiments of the present disclosure can be suitably used for a methane production apparatus that produces methane through the reaction between carbon dioxide and hydrogen.

### Reference Signs List

- **1**: honeycomb-like base material
- **12**: partition wall
- **13**: cell
- **14**: gas flow passage
- **15**: heat-generating portion
- **16**: insulating portion
- **2**: catalyst layer
- **100**: honeycomb structural body

## Claims

1. A honeycomb structural body, comprising:
a honeycomb-like base material including a partition wall that defines a plurality of cells, each including a gas flow passage; and
catalyst layers containing a methanation reaction catalyst for methanation of carbon dioxide through a reaction with hydrogen, the catalyst layers being provided on a surface of the partition wall so as to face the gas flow passages, respectively,
wherein the honeycomb-like base material includes a heat-generating portion configured to generate heat by application of a voltage.

2. The honeycomb structural body according to claim 1, wherein the heat-generating portion is formed of an electrically conductive ceramic material.

3. The honeycomb structural body according to claim 2, wherein the electrically conductive ceramic material contains Si and SiC.

4. The honeycomb structural body according to claim 3, wherein an oxide coating is formed on a surface of a combination of Si and SiC.

5. The honeycomb structural body according to claim 1,
wherein the honeycomb-like base material further includes an insulating portion formed of an insulating ceramic material, and
wherein the heat-generating portion is positioned on an upstream side of the insulating portion in a direction of passage of a fluid through the gas flow passages.

6. A methane production apparatus, comprising the honeycomb structural body of any one of claims 1 to 5.

7. A method of producing methane, comprising:
a step of causing the heat-generating portion of the honeycomb-like base material included in the honeycomb structural body of any one of claims 1 to 5 to generate heat at 200°C or more and 500°C or less by applying a voltage to the heat-generating portion; and
a step of supplying a raw material gas containing carbon dioxide and hydrogen to the gas flow passages after the heat-generating portion generates heat.
